# FASCICULE DE BREVET EUROPEEN

(11) **EP 0 971 589 B1**
(45) Date de publication et mention de la délivrance du brevet: **14.05.2003**
(21) Numéro de dépôt: 98917230.9
(22) Date de dépôt: 25.03.1998
(51) Int. Cl.: A01N 47/02, A01N 43/56, A01N 51/00

(54) **PROCEDE ET MOYENS D'ERADICATION DES PUCES DANS LES LOCAUX HABITES PAR LES PETITS MAMMIFERES**
BEKÄMPFUNGSVERFAHREN UND MITTEL GEGEN FLÖHE AN AUFENTHALTSPLÄTZEN KLEINER SÄUGER
PROCESS AND MEANS FOR THE ERADICATION OF FLEAS IN THE HABITATS OF SMALL MAMMALS

(30) Priorité: 26.03.1997 FR 9703709
(43) Date de publication de la demande: 19.01.2000
(73) Titulaire: MERIAL, 69002 Lyon (FR)
(72) Inventeur: HUNTER, James, S., Athens, GA 30605 (US); ETCHEGARAY, Jean-Pierre, F-31100 Toulouse (FR); JULIA, Bruno, F-31300 Toulouse (FR); JEANNIN, Philippe, F-31170 Tournefeuile (FR)
(74) Mandataire: Bernasconi, Jean
(86) Numéro de dépôt international: FR9800601
(87) Numéro de publication internationale: WO98042191

(56) Documents cités:
- EP-A- 0 214 477
- EP-A- 0 255 803
- EP-A- 0 295 117
- EP-A- 0 516 590
- EP-A- 0 682 869
- WO-A-96/16544
- DE-A- 4 443 888
- DE-A- 19 519 007
- FR-A- 2 713 889
- DATABASE CROPU STN-International STN-accession no. 97-89205, N.J.MEO ET AL.: "A comparison of the flea control efficacy of Frontline Spray treatment against the flea infestation prevention pack (VET-KEM) in the dog and cat (Interim Report)" XP002048645 & PROC.AM.ASSOC.VET.PARASITOL. (41 MEET.), 1996, page 52

## Description

La présente invention a trait à un procédé pour l'éradication de parasites, à savoir les parasites de l'ordre des Siphonaptères, notamment les puces, telles que, par exemple, Ctenocephalides felis et canis, mais également les autres puces de petits mammifères tels que, par exemple, lapins ou animaux de laboratoire.

Le contrôle des parasites de petits mammifères domestiques, par exemple chiens et chats, et notamment des puces, est connu pour être extrêmement difficile.

Généralement on cherche à contrôler les animaux eux-mêmes, soit à l'aide de colliers anti-puces contenant des insecticides divers, soit par application topique de préparations à base d'insecticides.

Néanmoins les puces restent toujours présentes dans l'environnement de l'animal, et notamment dans les locaux des animaux de compagnie, tels que les locaux domestiques, les chenils ou chatteries, ainsi que les laboratoires possédant des animaux.

L'éradication des puces dans ces locaux à l'aide de pesticides ou d'agents chimiques de traitement des locaux est une opération lourde et, sauf à laisser en permanence le local recouvert d'une substance insecticide, qui peut, à la longue, présenter une certaine toxicité, la réinfestation survient rapidement.

Il ne reste donc qu'à traiter régulièrement les animaux à l'aide d'insecticides présentant une durée d'efficacité aussi grande que possible, afin de réduire la périodicité et le coût des traitements.

Ainsi on a proposé récemment l'utilisation, pour le traitement des puces et des tiques chez les petits animaux, de préparations topiques sous la forme de préparations à pulvériser ou de préparations concentrées à application cutanée ponctuelle (spot on) dont le principe actif est constitué par le 1-[2,6-Cl₂ 4-CF₃ phényl]3-CN 4-[SO-CF₃]5-NH₂ pyrazole, dont la dénomination commune est fipronil.

En effet, les composés appartenant aux familles de pyrazoles, notamment de phénylpyrazole, décrites dans les brevets EP-A-295 217 et EP-A-352 944 se sont avérés extrêmement efficaces sur les puces.

La durée d'efficacité anti-puces du fipronil, sous forme de solution concentrée à application ponctuelle, dite spot on, peut dépasser 2 à 3 mois chez le chien et six semaines chez le chat.

Compte tenu de ces performances, les utilisateurs sont naturellement incités à allonger les périodes entre deux applications de façon à bénéficier de cet effet de longue durée.

Une explication possible de la longue durée d'activité sur l'animal peut être liée à la constatation que le fipronil se dissout dans le sébum et les glandes sudoripares pour être relargué pendant une longue durée.

Dans une communication (Meo N.J. et al. ; Proc. Am. Assoc. Vet. Parasitol. (41 Meet., 52, 1996)) il a été fait part d'un accroissement notable du pourcentage de non réapparition de puces dans des locaux non traités, si les animaux fréquentant ces locaux (chats, chiens) ont reçu des administrations mensuelles de pulvérisations (spray) du produit Frontline® Spray contenant du fipronil.

La présente invention se propose de simplifier et d'améliorer encore ce contrôle des puces.

L'invention a donc pour objet un procédé d'éradication des puces dans les locaux d'habitations ou d'hébergement de mammiferes de petite taille, notamment chats et chiens, caractérisé en ce que l'on applique périodiquement sur l'animal ou les animaux du local considéré, une préparation topique concentrée à application ponctuelle, de type spot on, en quantité efficacement parasiticide comprise entre 0,3 et 60 mg par kg de poids corporel, d'un composé de formule I ou, éventuellement, de formule II selon une périodicité mensuelle.

La formule I est la formule suivante : 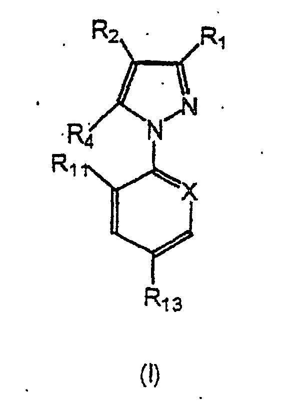 dans laquelle :
R₁ est CN ou méthyle ou un atome d'halogène ;
R₂ est S(O)ₙR₃ ou 4,5-dicyanoimidazol 2-yl ou haloalkyle ;
R₃ est alkyle ou haloalyle ;
R₄ représente un atome d'hydrogène ou d'halogène ; ou un radical NR₅R₆, S(O)ₘR₇, C(O)-R₇, C(O)O-R₇, alkyle, haloalkyle ou OR₈ ou un radical -N=C(R₉)(R₁₀) ;
R₅ et R₆ représentent indépendamment l'atome d'hydrogène ou un radical alkyle, haloalkyle, C(O)alkyle, alcoxycarbonyl, S(O)ᵣCF₃ ; ou R₅ et R₆ peuvent former ensemble un radical alkylène divalent qui peut être interrompu par un ou deux hétéroatomes divalents, tels que l'oxygène ou le soufre ;
R₇ représente un radical alkyle ou haloalkyle ;
R₈ représente un radical alkyle, haloalkyle ou un atome d'hydrogène ;
R₉ représente un radical alkyle ou un atome d'hydrogène ;
R₁₀ représente un groupe phényl ou hétéroalkyle éventuellement substitué par un ou plusieurs atomes d'halogène ou groupes tels que OH, -O-alkyle, -S-alkyle, cyano, ou alkyle ;
R₁₁ et R₁₂ représentent, indépendamment l'un de l'autre, un atome d'hydrogène ou d'halogène, ou éventuellement CN ou NO₂ ;
R₁₃ représente un atome d'halogène ou un groupe haloalkyle, haloalkoxy, S(O)_{q}CF₃ ou SF₅ ;
m, n, q, r représentent, indépendamment l'un de l'autre, un nombre entier égal à 0, 1 ou 2 ;
X représente un atome d'azote trivalent ou un radical C-R₁₂, les trois autres valences de l'atome de carbone faisant partie du cycle aromatique ;
sous réserve que, lorsque R₁ est méthyle, alors R₃ est haloalkyle, R₄ est NH₂, R₁₁ est Cl, R₁₃ est CF₃, et X est N ; ou lorsque R₂ est 4,8-dicyanoimidazol 2-yl, R₄ est Cl, R₁₁ est Cl, R₁₃ est CF₃ et X est =C-Cl.

La formule II est la formule suivante : 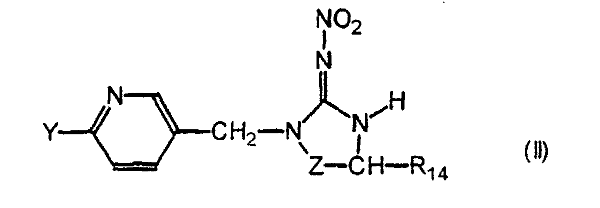 où Y est hydrogène ou halogène
R₁₄ est hydrogène ou méthyle
et Z est -(CH₂)ₙ- avec n = 1 ou 2.

De préférence, dans la formule (I),
R₁ est CN ou méthyle ;
R₂ est S(O)ₙR₃ ;
R₃ est haloalkyle ou éthyle
R₄ représente un atome d'hydrogène ou d'halogène ; ou un radical NR₅R₆, S(O)ₘR₇, C(O)R₇, alkyle, haloalkyle ou OR₈ ou un radical -N=C (R₉) (R₁₀) ;
R₅ et R₆ représentent indépendamment l'atome d'hydrogène ou un radical alkyle, haloalkyle, C(O)alkyle, S(O)ᵣCF₃ ; ou R₅ et R₆ peuvent former ensemble un radical alkylène divalent qui peut être interrompu par un ou deux hétéroatomes divalents, tels que l'oxygène ou le soufre ;
R₁₁ et R₁₂ représentent, indépendamment l'un de l'autre, un atome d'hydrogène ou d'halogène ;
sous réserve que, lorsque R₁ est méthyle, alors R₃ est haloalkyle, R₄ est NH₂, R₁₁ est Cl, R₁₃ est CF₃, et X est N.

On retiendra tout particulièrement les composés de formule (I) dans lesquels R₁ est CN. On retiendra aussi les composés dans lesquels R₁₃ est haloalkyle, de préférence CF₃ ou R₂ est S(O)ₙR₃ avec R₃ étant haloalkyle ou X = C-R₁₂, R₁₂ étant un atome d'halogène. On préfère aussi que R₁₁ soit un atome d'halogène.

Une classe préférée de composés de formule (I) est constituée par les composés tels que R₁ est CN, R₃ est haloalkyle, R₄ est NH₂, R₁₁ et R₁₂ sont indépendamment l'un de l'autre un atome d'halogène, et/ou R₁₃ est haloalkyle.

Les radicaux alkyle de la définition des composés de formules (I) comprennent généralement de 1 à 6 atomes de carbone. Le cycle formé par le radical alkylène divalent représentant R₅ et R₆ ainsi que l'atome d'azote auxquels R₅ et R₆ sont rattachés, est généralement un cycle à 5, 6 ou 7 chaînons.

Un composé de formule (I) tout particulièrement préféré dans l'invention est le
1-[2,6-Cl₂ 4-CF₃ phényl]3-CN 4-[SO-CF₃]5-NH₂ pyrazole, dénommé ci-après fipronil.

Parmi de nombreux autres composés avantageux on peut citer le 1-[2,6-Cl₂ 4-CF₃ phényl]3-CN4-[SO-C₂H₅]5-NH₂ pyrazole.

La préparation de composés de formule (I) peut être faite selon l'un ou l'autre des procédés décrits dans les demandes de brevet WO-A-87/3781, 93/6089, 94/21606 ou européenne EP-A-0 295 117, ou tout autre procédé relevant de la compétence de l'homme du métier spécialiste de synthèse chimique. Pour la réalisation chimique des produits de l'invention, l'homme de l'art est considéré comme ayant à sa disposition, entre autres, tout le contenu des "Chemical Abstracts" et des documents qui y sont cités.

De préférence dans le composé de formule (II) Y=Cl, R₁₄ = H et n = 1, c'est-à-dire le
1-[(6-chloro-3-pyridinyl)méthyl]4,5 dihydro-N-nitro-1H-imidazol-2-amine ou imidacloprid.

Les composés de formule (II) peuvent être préparés par les procédés correspondants décrits, par exemple, dans EP-A-0 192 060.

Par périodicité mensuelle on entend idéalement un traitement tous les mois mais on comprend que l'invention peut être mise en oeuvre à une fréquence supérieure, par exemple bihebdomadaire ou toutes les trois semaines, ou éventuellement, mais de façon non préférée, légèrement supérieure, par exemple toutes les cinq semaines.

La périodicité préférée est la périodicité mensuelle, une fréquence plus grande aboutissant à une consommation inutile. De plus la fréquence mensuelle a l'avantage de permettre à l'utilisateur de mémoriser et de programmer les applications.

Lorsque le local comporte plusieurs animaux, il est préférable, et plus simple, de traiter tous les animaux en même temps.

La quantité efficacement parasiticide, au sens de l'invention, est la quantité utilisée pour éradiquer les puces sur l'animal lui-même et peut donc correspondre aux doses déjà préconisées pour le traitement topique de l'animal pour les formulations déjà utilisées commercialement. Une telle dose doit pouvoir protéger l'animal lui-même pendant une durée d'au moins un mois.

La dose de composé actif est, de préférence comprise entre 5 et 15 mg par kilo de poids corporel par animal traité.

Le traitement selon l'invention peut être conduit de façon permanente, en tenant compte éventuellement des saisons d'infestation là où l'infestation est saisonnière. Un tel traitement permanent est préféré pour les locaux où se produisent de nombreuses entrées d'animaux, par exemple les élevages, chenils, chatteries ou cliniques vétérinaires.

On préférera, de façon particulièrement préférée, les préparations concentrées à l'application ponctuelle de type "spot on" formulées pour que le volume de préparation appliqué sur l'animal soit de l'ordre de 0,3 à 1 ml, de préférence 0,5 ml pour le chat et de l'ordre de 0,3 à 3 ml pour le chien en fonction du poids de l'animal.

Cette préparation peut comporter, outre le principe actif lui-même, un inhibiteur de cristallisation, un solvant organique et un cosolvant organique.

De préférence, le composé actif, notamment le composé de formule I, peut être présent dans la formulation à raison d'une concentration de 1 à 20 % et de préférence de 5 à 15 % (pourcentage en poids par volume).

L'objet du procédé selon l'invention peut être non thérapeutique, s'agissant d'une part de nettoyer les poils et la peau des animaux en éliminant les parasites présents et en évitant leurs résidus et déjections pour qué l'animal présente un pelage agréable à l'oeil et au toucher, s'agissant également de supprimer l'apparition et le développement de puces dans le local habité par l'animal.

L'objet peut aussi être thérapeutique lorsqu'il s'agit de traiter une parasitose ayant des conséquences pathogènes.

Les compositions concentrées pour application ponctuelle peuvent avantageusement comprendre :
a) le composé de formule I
b) un inhibiteur de cristallisation, notamment présent à raison de 1 à 20 % (P/V), de préférence de 5 à 15 %, cet inhibiteur répondant au test selon lequel :
   0,3 ml d'une solution comprenant 10 % (P/V) du composé de formule (I) dans le solvant d,fini sous c) ci-après, ainsi que 10 % de cet inhibiteur, sont déposés sur une lame de verre à 20°C pendant 24 heures, à la suite de quoi on observe à l'oeil nu peu ou pas de cristaux, notamment moins de 10 cristaux, de préférence 0 cristaux, sur la lame de verre,
c) un solvant organique ayant une constante diélectrique comprise entre 10 et 35, de préférence entre 20 et 30, la teneur de ce solvant c) dans la composition globale représentant de préférence le complément à 100 % de la composition,
d) un cosolvant organique ayant un point d'ébullition inférieur à 100°C, de préférence inférieur à 80°C et ayant une constante diélectrique comprise entre 10 et 40, de préférence entre 20 et 30 ; ce cosolvant peut avantageusement être présent dans la composition selon un ratio poids/poids (P/P) de d)/c) compris entre 1/15 et 1/2. Le solvant est volatil afin de servir notamment de promoteur de séchage et est miscible à l'eau et/ou au solvant c).

Quoique ceci ne soit pas préféré, la composition pour application ponctuelle peut éventuellement comprendre de l'eau, notamment à raison de 0 à 30 % (volume par volume V/V), en particulier de 0 à 5 %.

La composition à application ponctuelle peut aussi comprendre un agent antioxydant destiné à inhiber l'oxydation à l'air, cet agent étant notamment présent à raison de 0,005 à 1 % (P/V), de préférence de 0,01 à 0,05 %.

Les compositions selon l'invention destinées à des animaux de compagnie notamment chiens et chats, sont appliquées par dépôt cutané (en anglais "spot on") ; il s'agit généralement d'une application localisée sur une zone de surface inférieure à 10 cm², notamment comprise entre 5 et 10 cm², en particulier en deux points et de préférence localisée entre les épaules de l'animal. Après dépôt, la composition diffuse, notamment sur tout le corps de l'animal, puis sèche, sans cristalliser ni modifier l'aspect (notamment absence de tout dépôt blanchâtre ou d'aspect poussiéreux) ni le toucher du pelage.

Les compositions à application ponctuelle selon l'invention sont particulièrement avantageuses par leur efficacité, leur rapidité d'action, ainsi que par l'aspect agréable du poil des animaux après application et séchage.

Comme solvant organique c) utilisable dans l'invention on peut citer en particulier : l'acétone, l'acétonitrile, l'alcool benzylique, le butyldiglycol, le diméthylacétamide, le diméthylformamide, l'éther n-butylique du dipropylèneglycol, l'éthanol, l'isopropanol, le méthanol, l'éthylèneglycol monothyléther, l'éthylèneglycol monométhyléther, le monométhylacétamide, le monométhyléther de dipropylène glycol, les polyoxyéthylèneglycols liquides, le propylèneglycol, la 2-pyrrolidone, notamment la N-méthyl pyrrolidone, le monoéthyléther de diéthylèneglycol, l'éthylèneglycol, le diéthyphtalate, ou un mélange d'au moins deux d'entre eux.

Comme inhibiteur de cristallisation b) utilisable dans l'invention, on peut citer en particulier :
- la polyvinylpyrrolidone, les alcools polyvinyliques, les copolymères d'acétate de vinyle et de vinylpyrrolidone, les polyéthylèneglycols, l'alcool benzylique, le mannitol, le glycérol, le sorbitol, les esters de sorbitane polyoxyéthylénés ; la lécithine, la carboxyméthylcellulose sodique, les dérivés acryliques tels que métahcrylates et autres,
- les tensioactifs anioniques tels que les stéarates alcalins, notamment de sodium, de potassium ou d'ammonium ; le stéarate de calcium ; le stéarate de triéthanolamine ; l'abiétate de sodium ; les sulfates d'alkyle, notamment le laurylsulfate de sodium et le cétylsulfate de sodium ; le dodécylbenzènesulfonate de sodium, le dioctylsulfosuccinate de sodium ; les acides gras, notamment ceux dérivés de l'huile de coprah,
- les tensioactifs cationiques tels que les sels d'ammonium quaternaires hydrosolubles de formule N⁺R'R"R'"R"",Y⁻ dans laquelle les radicaux R sont des radicaux hydrocarbonés, éventuellement hydroxylés, et Y⁻ est un anion d'un d'acide fort tel que les anions halogénure, sulfate et sulfonates ; le bromure de cétyltriméthylammonium fait partie des tensioactifs cationiques utilisables,
- les sels d'amine de formule N⁺R'R"R"' dans laquelle les radicaux R sont des radicaux hydrocarbonés, éventuellement hydroxylés ; le chlorhydrate d'octadécylamine fait partie des tensioactifs cationiques utilisables,
- les tensioactifs non ioniques tels que les esters de sorbitane, éventuellement polyoxyéthylénés, en particulier Polysorbate 80, les éthers d'alkyle polyoxyéthylénés ; le stéarate de polyéthylèneglycol, les dérivés polyoxyéthylénés de l'huile de ricin, les esters de polyglycérol, les alcools gras polyoxyéthylénés, les acides gras polyoxyéthylénés, les copolymères d'oxyde d'éthylène et d'oxyde de propylène,
- les tensioactifs amphotères tels que les composés lauryle substitués de la betaïne, ou de préférence un mélange d'au moins deux d'entre eux.

De manière particulièrement préférée, on utilisera un couple inhibiteur de cristallisation, à savoir la combinaison d'un agent tensio-actif. Ces agents seront notamment choisis parmi les composés cités comme inhibiteur de cristallisation b).

Parmi les agents filmogènes de type polymérique particulièrement intéressants, on peut citer :
- les différents grades de polyvinylpyrrolidone,
- les alcools polyvinyliques, et
- les copolymères d'acétate de vinyle et de vinylpyrrolidone.

Pour ce qui est des agents tensio-actifs, on citera tout particulièrement les tensioactifs non ioniques, de préférence les esters de sorbitate polyoxyéthylénés et notamment les différents grades de Polysorbates, par exemple le Polysorbate 80.

Agent filmogène et agent tensioactif pourront notamment être incorporés en quantités proches ou identiques dans la limite des quantités totales d'inhibiteur de cristallisation mentionnées par ailleurs.

Le couple ainsi constitué assure de manière remarquable les objectifs d'absence de cristallisation sur le poil et de maintien de l'aspect cosmétique du pelage, c'est-à-dire sans tendance au collage ou à l'aspect poisseux, malgré la forte concentration en matière active.

Comme cosolvant d), on peut citer en particulier : l'éthanol absolu, l'isopropanol, le méthanol.

Comme agent antioxydant, on utilise notamment les agents classiques tels que : butylhydroxyanisole, butylhydroxytoluène, acide ascorbique, métabisulfite de sodium, gallate de propyle, thiosulfate de sodium, mélange d'au plus deux d'entre eux.

Les compositions pour application ponctuelle selon l'invention se préparent habituellement par simple mélange des constituants tels que précédemment définis ; de manière avantageuse, on commence par mélanger la matière active dans le solvant principal, et on ajoute ensuite les autres ingrédients ou adjuvants.

De façon avantageuse on peut prévoir des compositions prêtes à l'emploi, dosées pour des animaux de 1-10, 10-20, 20-40 kg respectivement.

De façon particulièrement préférée, la composition selon l'invention peut se présenter sous forme de solution, suspension ou émulsion concentrée pour une application ponctuelle sur une petite zone cutanée de l'animal, généralement entre les deux épaules (solution de type spot-on).

Le procédé selon l'invention peut également comporter, en plus, l'administration d'un autre parasiticide, de préférence à la même fréquence, et de préférence administré concomitamment, et préférentiellement à l'aide d'une composition unique comprenant un mélange ou une association de ce parasiticide et d'un insecticide de formule I ou II. Ces parasiticides associés ne sont pas connus pour avoir en eux-mêmes une activité permettant d'agir directement sur les insectes dans leur stade éloigné de l'animal, mais ils peuvent être utiles pour accroître encore l'efficacité contre les puces installées sur l'animal et également pour réduire les éventuels risques de survenue de résistances à l'insecticide.

Parmi ces parasiticides associés on peut citer notamment les composés mimant les hormones juvéniles, notamment :
azadirachtin - Agridyne
diofenolan (Ciba Geigy)
fenoxycarb (Ciba Geigy)
hydroprene (Sandoz)
kinoprene (Sandoz)
methoprene (Sandoz)
pyriproxyfene (Sumitomo/Mgk)
tetrahydroazadirachtin (Agridyne)
et le 4-chloro-2-(2-chloro-2-méthylpropyl)-5-(6-iodo-3-pyridylméthoxy)pyridizine-3(2H)-one et les inhibiteurs de la synthèse de la chitine, notamment :
   chlorfluazuron (Ishihara Sangyo)
   cyromazine (Ciba Geigy)
   diflubenzuron (Solvay Duphar)
   fluazuron (Ciba Geigy)
   flucycloxuron (Solvay Duphar)
   flufenoxuron (Cyanamid)
   hexaflumuron (Dow Elanco)
   lufenuron (Ciba Geigy)
   novaluron (Isagro, Italie)
   tebufenozide (Rohm & Haas)
   teflubenzuron (Cyanamid)
   triflumuron (Bayer)
ces composés étant définis par leur dénomination commune internationale (The Pesticide Manual, 10th edition, 1994, Ed. Clive Tomlin, Grande-Bretagne).

On peut citer, également comme inhibiteurs de synthèse de la chitine des composés tels que le 1-(2,6-difluorobenzoyl)-3-(2-fluoro-4-(trifluorométhyl)phényl-urée, le 1-(2,6-difluorobenzoyl)-3-(2-fluoro-4-(1,1,2,2-tétrafluoroéthoxy)phénylurée, le 1-(2,6-difluorobenzoyl)-3-(2-fluoro-4-trifluorométhyl)phénylurée.

Les composés associés préférés sont les méthoprènes, pyriproxyphènes, hydroprène, cyromazine, lufénuron, novaluron, et le 1-(2,6-difluorobenzoyl)-3-(2-fluoro-4-(trifluorométhyl)phénylurée.

On préfère que l'administration des deux types de composés soit concomitante et de préférence simultanée.

De préférence, le traitement est conduit de façon à administrer à l'animal une dose de 0,1 à 40 et notamment de 1 à 20 mg/kg pour le dérivé de formule I ou II et une dose de 0, 1 à 40, notamment 1 à 30 mg/kg pour le composé associé.

Les doses préférées sont de 5 à 15 mg/kg pour le composé de formule 1 et de 0,5 à 15 mg/kg pour les composés associés.

Les proportions en poids du composé de formule I ou II et du composé associé sont, de préférence, comprises entre 80/20 et 20/80.

Dans une autre forme de réalisation le parasiticide associé peut être un parasiticide endectocide de type lactone macrocyclique.

De préférence, ce parasiticide est choisi dans le groupe formé par les avermectines, l'ivermectine, l'abamectine, la doramectine, la moxydectine, les milbémycines et les dérivés de ces composés.

La structure, les caractéristiques et les procédés de fabrication de ces composés sont bien connus de l'homme de l'art et on pourra se référer à la littérature technique et commerciale largement disponible. Pour les avermectines, l'ivermectine et l'abamectine, on peut se référer, par exemple, à l'ouvrage "Ivermectin and Abamectin", 1989 par M.H. FISCHER and H. MROZIK, William C. CAMPBELL, ed. Springer Verlag. ou ALBERS-SCH™NBERG et al. (1981) "Avermectins Structure Determination". J. Am. Chem. Soc. 103: 4216-4221. Pour la doramectine on pourra consulter notamment "Veterinary Parasitology" vol. 49 no 1, juillet 1993: 5-15. Pour les milbémycines on pourra se référer, entre autres, à DAVIES H.G. et al., 1986, "Avermectins and Milbemycins". Nat. Prod. Rep. 3: 87-121, Mrozik H. et al, 1983, Synthesis of milbemycins from avermectins, Tetrahedron lett. 24: 5333-5336 ainsi que US-A-4 134 973.

L'administration des deux types de composés peut être concomitante et de préférence simultanée sous forme d'une composition unique.

La quantité efficace de composé endectocide est de préférence comprise entre 0,1 µg, préférentiellement 1 µg et 1 mg, et de façon particulièrement préférée 5 à 200 µg/kg de poids d'animal. Les proportions en poids entre le composé de formule (I) ou (II) et le composé endectocide sont de préférence comprises entre 5/1 et 10 000/1.

Les mécanismes par lequel les puces disparaissent des locaux eux-mêmes ne sont pas bien connus et l'efficacité de l'invention est particulièrement surprenante.

La présente invention a également pour objet des ensembles conditionnés ou kits comprenant une ou plusieurs unités de compositions utilisables dans l'invention représentant une pluralité de doses mensuelles destinées à être successivement administrées à un animal pour la mise en oeuvre du procédé pendant une longue durée, par exemple, suivant les pays, pendant une durée d'un an ou pendant une durée d'une saison d'infestation des puces.

De préférence, ce kit comporte une pluralité de doses unitaires mensuelles distinctes, par exemple 3 ou 6 (pour une saison) ou 12 (pour un an) doses distinctes contenues dans autant de récipients de type spot on. Dans cette forme de réalisation on prévoit plusieurs versions de kit en fonction du poids des animaux.

Le kit comprend également des moyens d'explication tels qu'une notice pour assurer la périodicité de l'utilisation des doses.

Dans une autre forme de réalisation les différentes doses peuvent être contenues dans un récipient unique muni de moyens pour assurer l'application d'une dose correspondant à la quantité destinée à être administrée mensuellement. Ce moyen peut être, par exemple, une pompe ou valve doseuse assurant la délivrance d'une dose précise. Il peut aussi, plus simplement, être formé d'une graduation au regard d'une surface transparente de récipient, permettant de connaître le volume liquide de composition qui reste dans le récipient.

L'invention a également pour objet l'utilisation d'un composé de formule I ou de formule II pour la préparation d'une composition pour l'éradication des puces dans les locaux d'habitation ou d'hébergement des mammifères de petites tailles, notamment chats et chiens, par application périodique sur l'animal ou les animaux du local considéré d'une préparation topique définie ci-dessus en quantité efficacement parasiticide d'un composé de formule I, ou éventuellement de formule II selon une périodicité mensuelle.

D'autres avantages et caractéristiques de l'invention apparaîtront à la lecture de la description suivante, faite à titre d'exemple non limitatif.

### Exemple 1

L'essai a utilisé des chiens en bonne santé.

Les chiens ont été répartis par groupe de trois chiens dans des enclos de surface environ 4,80 m de long (16 pieds) et 1,5 m de large (5 pieds) contenant chacun un abri et un sol spécialement favorable au développement des stades immatures des puces (Ctenocephalides felis). Tous les chiens ont été volontairement et identiquement préinfestés puis régulièrement infestés par des puces à un rythme tel que le nombre total des puces portées par chaque animal du groupe témoin A n'est pas descendu significativement au-dessous de 20.

Un total de 18 chiens, à savoir six enclos, a été attribué au groupe témoin A.

Un groupe C de six chiens (2 enclos) ainsi préinfestés a reçu le traitement conformément à l'invention par application d'une solution ponctuelle cutanée ayant une concentration de 10 % en fipronil.

Les doses de composition concentrée à application cutanée locale de fipronil ont été les suivantes :

| poids de l'animal | doses |
|---|---|
| 5-10 kg | 0,67 ml |
| 10-20 kg | 1,34 ml |
| 20-40 kg | 2,68 ml |

Les animaux du groupe C ont été traités le jour 0 et ensuite tous les 28 jours, soit les jours 28, 56 et 86.

Les vérifications (comptage hebdomadaire des oeufs, comptage sur corps les semaines 1, 3, 5, 7, 9 et comptage au peigne les semaines 2, 4, 6, 8, 10, 11, 12) ont montré un taux de contrôle de l'infestation égal à 100 %.

Un essai similaire sur chats a donné les mêmes résultats, à savoir une absence totale de réinfestation.

## Revendications

1. Utilisation d'un composé de formule I ou de formule II pour la préparation d'une composition pour l'éradication des puces dans les locaux d'habitation ou d'hébergement des mammifères de petites tailles, notamment chats et chiens, par application périodique sur l'animal ou les animaux du local considéré d'une préparation topique concentrée à application ponctuelle, en quantité efficacement parasiticide comprise entre 0,3 et 60 mg par kg de poids corporel, d'un composé de formule I, ou éventuellement de formule II selon une périodicité mensuelle. 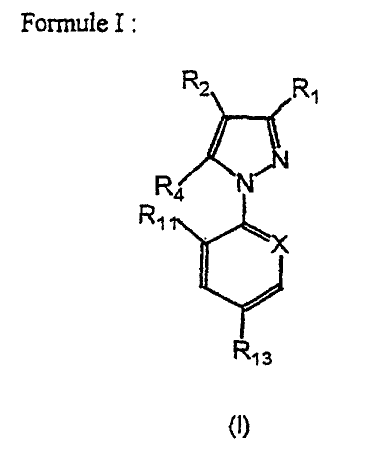 dans laquelle :
R₁ est CN ou méthyle ou un atome d'halogène ;
R₂ est S(O)ₙR₃ ou 4,5-dicyanoimidazol 2-yl ou haloalkyle ;
R₃ est alkyle ou haloalyle ;
R₄ représente un atome d'hydrogène ou d'halogène ; ou un radical NR₅R₆, S(O)ₘR₇, C(O)-R₇, C(O)O-R₇, alkyle, haloalkyle ou OR₈ ou un radical -N=C(R₉)(R₁₀) ;
R₅ et R₆ représentent indépendamment l'atome d'hydrogène ou un radical alkyle, haloalkyle, C(O)alkyle, alcoxycarbonyl, S(O)ᵣCF₃ ; ou R₅ et R₆ peuvent former ensemble un radical alkylène divalent qui peut être interrompu par un ou deux hétéroatomes divalents, tels que l'oxygène ou le soufre ;
R₇ représente un radical alkyle ou haloalkyle ;
R₈ représente un radical alkyle, haloalkyle ou un atome d'hydrogène ;
R₉ représente un radical alkyle ou un atome d'hydrogène ;
R₁₀ représente un groupe phényl ou hétéroalkyle éventuellement substitué par un ou plusieurs atomes d'halogène ou groupes tels que OH, -O-alkyle, -S-alkyle, cyano, ou alkyle ;
R₁₁ et R₁₂ représentent, indépendamment l'un de l'autre, un atome d'hydrogène ou d'halogène, ou éventuellement CN ou NO₂ ;
R₁₃ représente un atome d'halogène ou un groupe haloalkyle, haloalkoxy, S(O)_{q}CF₃ ou SF₅ ;
m, n, q, r représentent, indépendamment l'un de l'autre, un nombre entier égal à 0, 1 ou 2 ;
X représente un atome d'azote trivalent ou un radical C-R₁₂, les trois autres valences de l'atome de carbone faisant partie du cycle aromatique ;
sous réserve que, lorsque R₁ est méthyle, alors R₃ est haloalkyle, R₄ est NH₂, R₁₁ est Cl, R₁₃ est CF₃, et X est N ; ou lorsque R₂ est 4,8-dicyanoimidazol 2-yl, R₄ est Cl, R₁₁ est Cl, R₁₃ est CF₃ et X est =C-Cl. 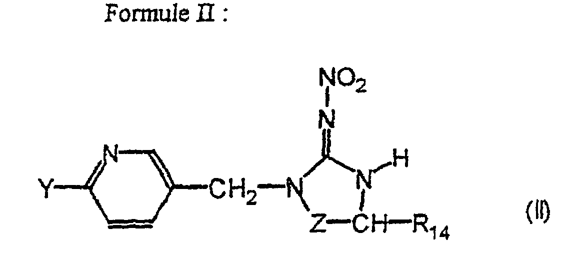 où
Y est hydrogène ou halogène
R₁₄ est hydrogène ou méthyle
et Z est -(CH₂)ₙ- avec n = 1 ou 2.

2. Utilisation selon la revendication 1 dans laquelle la quantité de composé de formule I ou éventuellement de formule II est comprise entre 5 et 15 mg/kg de poids corporel.

3. Utilisation selon l'une des revendications 1 et 2 dans laquelle le composé de formule I est présent à une concentration de 1 à 20% (poids/volume).

4. Utilisation selon la revendication 3 dans laquelle la concentration est de 5 à 15% (poids/volume).

5. Utilisation selon l'une des revendications 1 à 4, dans laquelle dans la formule
R₁ est CN ou méthyle ;
R₂ est S(O)ₙR₃ ;
R₄ représente un atome d'hydrogène ou d'halogène ; ou un radical NR₅R₆, S(O)ₘR₇, C(O)R₇, alkyle, haloalkyle ou OR₈ ou un radical -N=C (R₉) (R₁₀) ;
R₅ et R₆ représentent indépendamment l'atome d'hydrogène ou un radical alkyle, haloalkyle, C(O)alkyle, S(O)ᵣCF₃ ; ou R₅ et R₆ peuvent former ensemble un radical alkylène divalent qui peut être interrompu par un ou deux hétéroatomes divalents, tels que l'oxygène ou le soufre ;
R₁₁ et R₁₂ représentent, indépendamment l'un de l'autre, un atome d'hydrogène ou d'halogène ;
sous réserve que, lorsque R₁ est méthyle, alors R₃ est haloalkyle, R₄ est NH₂, R₁₁ est Cl, R₁₃ est CF₃, et X est N.

6. Utilisation selon la revendication 5 dans laquelle R₁ est CN.

7. Utilisation selon l'une des revendications 1 à 6 dans laquelle R₁₃ est haloalkyle, de préférence CF₃

8. Utilisation selon l'une des revendication 1 à 7 dans laquelle R₂ est S(O)ₙR₃ avec R₃ étant haloalkyle.

9. Utilisation selon l'une des revendications 1 à 8 dans laquelle X = C-R₁₂, R₁₂ étant un atome d'halogène.

10. Utilisation selon l'une des revendications 1 à 9 dans laquelle R₁ est CN, R₃ est haloalkyle, R₄ est NH₂, R₁₁ et R₁₂ sont indépendamment l'un de l'autre un atome d'halogène, et/ou R₁₃ est haloalkyle.

11. Utilisation selon l'une des revendications 1 à 4, dans laquelle le composé est le 1-[2,6-Cl₂ 4-CF₃ phényl]3-CN 4-[SO-CF₃]5-NH₂ pyrazole, dénommé fipronil.

12. Utilisation selon l'une des revendications 1 à 4 dans laquelle, dans le composé de formule (II), Y=Cl, R₁₄ = H et n = 1, c'est-à-dire le 1-[(6-chloro-3-pyridinyl)méthyl]4,5 dihydro-N-nitro-1H-imidazol-2-amine ou imidacloprid.

13. Utilisation selon l'une des revendications 1 à 12 pour un volume de préparation appliqué sur l'animal de l'ordre de 0,3 à 1 ml, pour le chat et de l'ordre de 0,3 à 3 ml pour le chien en fonction du poids de l'animal.

14. Utilisation selon l'une des revendications 1 à 13 pour une formulation comprenant outre le principe actif lui-même, un inhibiteur de cristallisation, un solvant organique et un cosolvant organique.

15. Utilisation selon l'une des revendications 1 à 14, pour la préparation d'une formulation comprenant en outre, un autre parasiticide.

16. Utilisation selon la revendication 15 dans laquelle cet autre parasiticide est choisi parmi les composés mimant les hormones juvéniles ou les inhibiteurs de la synthèse de la chitine.

17. Utilisation selon la revendication 16 dans laquelle ledit autre parasiticide est le méthoprène ou le pyriproxyfène.

18. Utilisation selon la revendication 15 dans laquelle cet autre parasiticide est un parasiticide endectocide de type lactone macrocyclique, notamment choisi dans le groupe formé par les avermectines, l'ivermectine, l'abamectine, la doramectine, la moxydectine, les milbémycines et les dérivés de ces composés.

19. Utilisation selon la revendication 18 dans laquelle la quantité dudit parasiticide endectocide est comprise entre 0,1 µg et 1 mg, et notamment de 5 à 200 µg, par kg de poids corporel.

20. Ensembles conditionnés ou kits comprenant une ou plusieurs unités de compositions préparées selon l'une des revendications 1 à 19, représentant une pluralité de doses mensuelles distinctes destinées à être successivement administrées à un animal pendant une longue durée, notamment pendant une durée d'un an ou pendant une durée d'une saison d'infestation des puces.

21. Ensembles ou kits selon la revendication 20, **caractérisés en ce qu'**ils comportent une pluralité de doses unitaires distinctes contenues dans autant de récipients de type spot on ou pour on.

22. Procédé non thérapeutique d'éradication des puces dans les locaux d'habitations ou d'hébergement de mammifères de petite taille, notamment chats et chiens, **caractérisé en ce que** l'on applique périodiquement sur l'animal ou les animaux du local considéré, selon une périodicité mensuelle, une composition préparée selon l'une des revendications 1 à 19.

23. Procédé selon a revendication 22, **caractérisé en ce que**, lorsque le local comporte plusieurs animaux, on traite tous les animaux en même temps.

24. Procedé selon l'une des revendications 22 et 23 **caractérisé en ce que** le traitement est conduit de façon permanente, en tenant compte éventuellement des saisons d'infestation là où l'infestation est saisonnière.

## Patentansprüche

1. Verwendung einer Verbindung mit der Formel I oder II für die Herstellung einer Zusammensetzung zur Vernichtung von Flöhen an Plätzen, wo kleine Säuger, insbesondere Katzen und Hunde, wohnen oder sich aufhalten, durch im monatlichen Abstand periodische Anwendung einer konzentrierten topischen Zubereitung für punktuelle Anwendung auf das Tier oder die Tiere des betreffenden Platzes in einer parasitentötend wirksamen Menge von 0,3 bis 60 mg pro kg Körpergewicht einer Verbindung mit der Formel I oder gegebenenfalls II: 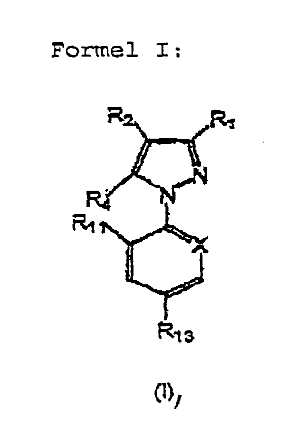 in welcher
- R₁ CN, einen Methylrest oder ein Halogenatom,
- R₂ S(O)ₙR₃, 4,5-Dicyanoimidazol-2-yl oder Halogenalkyl,
- R₃ einen Alkyl- oder Halogenalkylrest und
- R₄ ein Wasserstoff- bzw. Halogenatom, einen NR₅R₆-, S(O)ₘR₇-, C(O)-R₇-, C(O)O-R₇-, Alkyl-, Halogenalkyl- bzw. OR₈-Rest oder einen -N=C(R₉)(R₁₀)-Rest bedeutet und
- R₅ und R₆ unabhängig voneinander ein Wasserstoffatom oder einen Alkyl-, Halogenalkyl-, C(O)Alkyl-, Alkoxycarbonyl- und S(O)ᵣCF₃-Rest bedeuten oder R₅ und R₆ zusammen einen zweiwertigen Alkylenrest bilden können, der durch ein oder zwei zweiwertige Heteroatome wie Sauerstoff oder Schwefel unterbrochen sein kann, und
- R₇ einen Alkyl- oder Halogenalkylrest,
- R₈ einen Alkyl- und Halogenalkylrest oder ein Wasserstoffatom,
- R₉ einen Alkylrest oder ein Wasserstoffatom und
- R₁₀ einen Phenyl- oder Heteroalkylrest, der gegebenenfalls durch ein oder mehrere Halogenatome oder Gruppen wie OH, -O-Alkyl, -S-Alkyl, Cyano oder Alkyl substituiert ist, bedeutet und
- R₁₁ und R₁₂ unabhängig voneinander ein Wasserstoff- bzw. Halogenatom oder gegebenenfalls CN bzw. NO₂ bedeuten und
- R₁₃ ein Halogenatom oder einen Halogenalkyl-, Halogenalkoxy-, S(O)_{q}CF₃- bzw. SF₅-Rest bedeutet und
- m, n, q, r unabhängig voneinander eine ganze Zahl von 0, 1 oder 2 bedeuten und
- X ein dreiwertiges Stickstoffatom oder einen C-R₁₂-Rest bedeutet, wobei die drei anderen Wertigkeiten des Kohlenstoffatoms Teil des aromatischen Cyclus sind,
- unter der Voraussetzung, dass, wenn R₁ den Methylrest bedeutet, R₃ einen Halogenalkylrest, R₄ NH₂, R₁₁ Cl, R₁₃ CF₃ und X N bedeutet, oder, wenn R₂ 4,8-Dicyanoimidazol-2-yl bedeutet, R₄ Cl, R₁₁ Cl, R₁₃ CF₃ und X =C-Cl bedeutet, 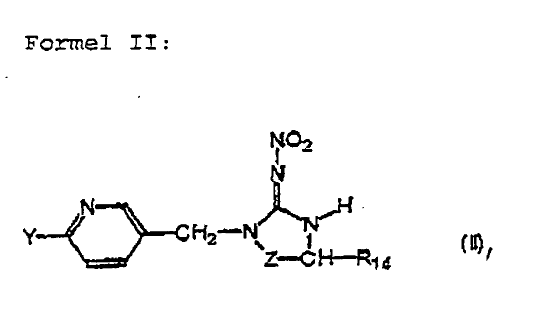
worin
- Y ein Wasserstoff- oder Halogenatom,
- R₁₄ ein Wasserstoffatom oder den Methylrest und
- Z -(CH₂)ₙ-, mit n = 1 oder 2, bedeutet.

2. Anwendung nach Anspruch 1, bei welcher die Menge an Verbindung mit der Formel I oder gegebenenfalls II 5 bis 15 mg/kg Körpergewicht beträgt.

3. Anwendung nach Anspruch 1 oder 2, bei welcher die Verbindung mit der Formel I mit einer Konzentration von 1 bis 20 % (Gew./Vol.) vorliegt.

4. Anwendung nach Anspruch 3, bei welcher die Konzentration 5 bis 15 % (Gew./Vol.) beträgt.

5. Anwendung nach einem der Ansprüche 1 bis 4, bei welcher in der Formel I
- R₁ CN oder den Methylrest,
- R₂ S(O)ₙR₃ und
- R₄ ein Wasserstoff- bzw. Halogenatom oder einen NR₅R₆-, S(O)ₘR₇-, C(O)R₇-, Alkyl- und Halogenalkylrest bzw. OR₈ oder einen -N=C(R₉) (R₁₀)-Rest bedeutet und
- R₅ und R₆ unabhängig voneinander ein Wasserstoffatom oder einen Alkyl-, Halogenalkyl-, C(O)Alkyl- und S(O)ᵣCF₃-Rest bedeuten oder R₅ und R₆ zusammen einen zweiwertigen Alkylenrest bilden können, der durch ein oder zwei zweiwertige Heteroatome wie Sauerstoff oder Schwefel unterbrochen sein kann, und
- R₁₁ und R₁₂ unabhängig voneinander ein Wasserstoffoder Halogenatom bedeuten,
- unter der Voraussetzung, dass, wenn R₁ einen Methylrest bedeutet, R₃ einen Halogenalkylrest, R₄ NH₂, R₁₁ Cl, R₁₃ CF₅ und X N bedeutet.

6. Anwendung nach Anspruch 5, bei welcher R₁ CN bedeutet.

7. Anwendung nach einem der Ansprüche 1 bis 6, bei welcher R₁₃ einen Halogenalkylrest und vorzugsweise CF₃ bedeutet.

8. Anwendung nach einem der Ansprüche 1 bis 7, bei welcher R₂ S(O)ₙR₃ mit R₃ als Halogenalkylrest bedeutet.

9. Anwendung nach einem der Ansprüche 1 bis 8, bei welcher X = C-R₁₂ und R₁₂ ein Halogenatom bedeutet.

10. Anwendung nach einem der Ansprüche 1 bis 9, bei welcher R₁ CN, R₃ einen Halogenalkylrest und R₄ NH₂ bedeutet und R₁₁ und R₁₂ unabhängig voneinander ein Halogenatom bedeuten und/oder R₁₃ einen Halogenalkylrest bedeutet.

11. Anwendung nach einem der Ansprüche 1 bis 4, bei welcher die Verbindung 1-[2,6-Cl₂-4-CF₃-phenyl]-3-CN-4-[SO-CF₃]-5-NH₂-pyrazol, das als Fipronil bezeichnet wird, ist.

12. Anwendung nach einem der Ansprüche 1 bis 4, bei welcher in der Verbindung mit der Formel II Y = Cl, R₁₄ = H und n = 1, d.h. 1-[(6-Chlor-3-pyridinyl)methyl]-4,5-dihydro-N-nitro-1H-imidazol-2-amin bzw. Imidacloprid ist.

13. Anwendung nach einem der Ansprüche 1 bis 12 mit einem auf das Tier anzuwendenden Volumen der Zubereitung von etwa 0,3 bis 1 ml für die Katze und etwa 0,3 bis 3 ml für den Hund in Abhängigkeit vom Körpergewicht des Tieres,

14. Anwendung nach einem der Ansprüche 1 bis 13 mit einer Formulierung, die außer dem Wirkstoff einen Kristallisationsinhibitor, ein organisches Lösungsmittel und ein organisches Colösungsmittel enthält.

15. Anwendung nach einem der Ansprüche 1 bis 14 zur Herstellung einer Formulierung, die außerdem ein weiteres Antiparasitikum enthält.

16. Anwendung nach Anspruch 15, bei welcher das weitere Antiparasitikum aus Verbindungen ausgewählt wird, welche die Wachstumshormone oder die Inhibitoren für die Chitinsynthese nachahmen.

17. Anwendung nach Anspruch 16, bei welcher das weitere Antiparasitikum Methopren oder Pyriproxyfen ist.

18. Anwendung nach Anspruch 15, bei welcher das weitere Antiparasitikum ein endektozides Antiparasitikum vom Typ makrocyclisches Lacton ist, das insbesondere aus der Gruppe ausgewählt wird, die von Avermectinen, Ivermectin, Abamectin, Doramectin, Moxydectin, Milbemycinen und den Derivaten dieser Verbindungen gebildet wird.

19. Anwendung nach Anspruch 18, bei welcher die Menge des endektoziden Antiparasitikums 0,1 µg bis 1 mg und insbesondere 5 bis 200 µg pro kg Körpergewicht beträgt.

20. Verpackungseinheiten oder Kits, die eine oder mehrere Einheiten von Zusammensetzungen enthalten, die nach einem der Ansprüche 1 bis 19 hergestellt sind, und eine Vielzahl monatlicher Einzeldosen darstellen, die vorgesehen sind, nacheinander über einen längeren Zeitraum, insbesondere über einen Zeitraum von einem Jahr oder des Befalls mit Flöhen, einem Tier verabreicht zu werden.

21. Verpackungseinheiten oder Kits nach Anspruch 20, **dadurch gekennzeichnet, dass** sie eine Vielzahl von jeweils in einem Behältnis enthaltenen Einzeldosen vom Typ Spot on oder Pour on umfassen.

22. Nichttherapeutisches Verfahren zur Vernichtung von Flöhen an Wohn- oder Aufenthaltsplätzen von kleinen Säugern, insbesondere Katzen und Hunden, **dadurch gekennzeichnet, dass** auf das Tier oder die Tiere des betreffenden Platzes eine nach einem der Ansprüche 1 bis 19 hergestellte Zusammensetzung periodisch im Monatsabstand angewendet wird.

23. Verfahren nach Anspruch 22, **dadurch gekennzeichnet, dass,** wenn sich an dem Platz mehrere Tiere aufhalten, sämtliche Tiere gleichzeitig behandelt werden.

24. Verfahren nach Anspruch 22 oder 23, **dadurch gekennzeichnet, dass** die Behandlung permanent durchgeführt wird, wobei gegebenenfalls Befallszeiträume berücksichtigt werden, wenn der Befall ein saisonaler ist.

## Claims

1. Use of a compound of formula I or formula II for the preparation of a composition for the eradication of fleas from the living or accommodation areas of small mammals, in particular cats and dogs, by the periodic spot-type application to the animal or animals from the area in question of a concentrated topical preparation in an effectively parasiticidic quantity in the range of between 0.3 and 60 mg per leg body weight, of a compound of formula I, or possibly of formula II at monthly intervals 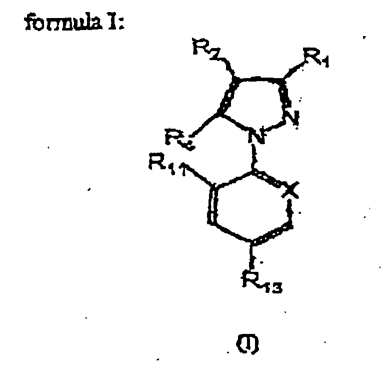 wherein
R₁ is CN or methyl or a halogen atom;
R₂ is S(O)nR₃ or 4,5-dicyanoimidazole 2-yl or haloalkyl;
R₃ is alkyl or haloalkyl;
R₄ represents a hydrogen or halogen, atom; or an NR₅R₆, S(O)ₘR₇, C(O)-R₇, C(O)O-R₇, alkyl, haloalkyl or OR₈ radical or an -N=C(R₉)(R₁₀) radical;
R₅ and R₆ independently represent the hydrogen atom or an alkyl, haloalkyl, C(O)alkyl, alcoxy carbonyl, S(O)CF₃ radical; or R₅ and R₆ can together form a divalent alkylene radical which can be broken by one or two divalent heteroatoms, such as oxygen or sulphur;
R₇ represents an alkyl or haloalkyl radical;
R₈ represents an alkyl, haloalkyl radical or a hydrogen atom;
R₉ represents an alkyl radical or a hydrogen atom;
R₁₀ represents a phenyl or heteroalkyl group possibly substituted by one or more halogen atoms or groups such as OH, -O-alkyl, -S-alkyl, cyano or alkyl groups;
R₁₁ and R₁₂ independently of one another represent a hydrogen or halogen atom, or possibly CN or NO₂;
R₁₃ represents a halogen atom or a haloalkyl, haloalkoxy group, S(O)_{q}CF₃ or SF₅;
m, n, q, r independently of one another represent a whole number equal to 0, 1 or 2;
X represents a trivalent nitrogen atom or a C-R₁₂ radical, the other three valencies of the carbon atom forming part of the aromatic cycle;
with the reservation that when R₁ is methyl, then R₃ is haloalkyl, R₄ is NH₂, R₁₁ is Cl, R₁₃ is CF₃ and X is N; or when R₂ is 4,8-dicyanoimidazole 2-yl, R₄ is Cl, R₁₁ is Cl, R₁₃ is CF₃ and X is =C-Cl 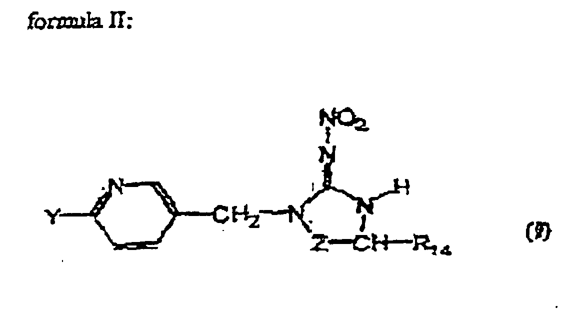 where
Y is hydrogen or halogen
R₁₄ is hydrogen or methyl
and Z is -(CH₂)ₙ- where n = 1 or 2.

2. Use according to Claim 1, wherein the quantity of the compound of formula I or possibly formula II is in the range of between 5 and 15 mg/kg body weight.

3. Use according to one of Claims 1 and 2, wherein the compound of formula I is present in a concentration of 1 to 20% (weight/volume).

4. Use according to Claim 3, wherein the concentration is 5 to 15% (weight/volume).

5. Use according to one of Claims 1 to 4, wherein in formula I
R₁ is CN or methyl;
R₂ is S(O)ₙR₃;
R₄ represents a hydrogen or halogen atom; or an NR₅R₆, S(O)ₘR₇, C(O)R₇, alkyl, haloalkyl or OR₈ radical or a -N=C(R₉)(R₁₀) radical;
R₅ and R₆ independently represent the hydrogen atom or an alkyl, haloalkyl, C(O)alkyl, S(O)ᵣCF₃ radical; or R₅ and R₆ can together form a divalent alkylene radical which can be broken by one or two divalent heteroatoms, such as oxygen or sulphur;
R₁₁ and R₁₂ independently of one another represent a hydrogen or halogen atom;
with the reservation that when R₁ is methyl, then R₃ is haloalkyl, R₄ is NH₂, R₁₁ is Cl, R₁₃ is CF₃ and X is N.

6. Use according to Claim 5, wherein R₁ is CN.

7. Use according to one of Claims 1 to 6, wherein R₁₃ is haloalkyl, preferably CF₃.

8. Use according to one of Claims 1 to 7, wherein R₂ is S(O)ₙR₃, where R₃ is haloalkyl,

9. Use according to one of Claims 1 to 8, wherein X = C-R₁₂, where R₁₂ is a halogen atom.

10. Use according to one of Claims 1 to 9, wherein R₁ is CN, R₃ is haloalkyl, R₄ is NH₂, R₁₁ and R₁₂ independently of one another are a halogen atom, and/or R₁₃ is haloalkyl.

11. Use according to one of Claims 1 to 4, wherein the compound is the 1-[2,6-Cl₂ 4-CF₃ phenyl]3-CN 4-[SO-CF₃]5-NH₂ pyrazole named fipronil.

12. Use according to one of Claims 1 to 4, wherein in the compound of formula (II), Y=Cl, R₁₄=H and n=1, i.e. the 1-[(6-chloro-3-pyridinyl)methyl]4,5 dihydro-N-nitro-1H-imidazole-2-amine or imidacioprid.

13. Use according to one of Claims 1 to 12 for a volume of preparation applied to the animal in the order of 0.3 to 1 ml for cats, and in the order of 0.3 to 3 ml for dogs, as a function of the body weight of the animal.

14. Use according to one of Claims 1 to 13 for a formulation comprising a crystallisation inhibitor, an organic solvent and an organic co-solvent, besides the active principle itself.

15. Use according to one of Claims 1 to 14 for the preparation of a formulation additionally containing another parasiticide.

16. Use according to Claim 15, wherein this other parasiticide is selected from compounds that mimic the juvenile hormones or inhibitors of chitin synthesis.

17. Use according to Claim 16, wherein said other parasiticide is methoprene or pyriproxyfen.

18. Use according to Claim 15, wherein this other parasiticide is a macrocyclic lactone-type endectocide parasiticide, in particular selected from the group formed by avermectins, ivermectin, abamectin, doramectin, moxidectin, milbemycins and the derivatives of these compounds.

19. Use according to Claim 18, wherein the quantity of said endectocide parasiticide is in the range of between 0.1 µg and 1 mg, and in particular 5 to 200 µg, per kg body weight.

20. Prepacked packs or kits containing one or more units of compositions prepared according to one of Claims 1 to 19, constituting a plurality of clearly defined monthly doses intended for successive administration to an animal over a long period, in particular over the period of a year or over a period of a flea infestation season.

21. Packs or kits according ro Claim 20, **characterised in that** they comprise a plurality of clearly defined specific doses contained in an appropriate number of receptacles of the spot-on or pour-on application type.

22. Non-therapeutic method for the eradication of fleas in living or accommodation areas of small mammals, in particular cats and dogs, **characterised in that** a composition prepared according to one of Claims 1 to 19 is applied periodically to the animal or animals of the area in question at monthly intervals.

23. Method according to Claim 22, **characterised in that** if the area contains several animals, all the animals are treated at the same time.

24. Method according to one of Claims 22 and 23, **characterised in that** the treatment is conducted on a permanent basis, possibly taking into consideration seasons of infestation in cases where infestation is seasonal.
